# EUROPEAN PATENT APPLICATION

(11) **EP 3 984 378 A1**
(43) Date of publication of application: **20.04.2022**
(21) Application number: 21158513.8
(22) Date of filing: 22.02.2021
(51) Int. Cl.: A23L 33/105, A23L 33/125, A23L 33/15, A23L 33/17, A23L 33/18, A23L 33/185, A23L 33/00, A23L 2/52, A23L 29/30, A61K 31/195, A61K 31/198, A61K 31/205, A61K 31/4415, A61K 31/455, A61K 31/51, A61K 31/525, A61K 31/714

(54) **DIETARY SUPPLEMENT FOR SPORT USE**

(30) Priority: 14.10.2020 IT 202000024256
(71) Applicant: Ovidia Food S.r.l., 36100 Vicenza (IT)
(72) Inventor: Bonetto, Davide, 36100 Vicenza (IT)
(74) Representative: Leganza, Alessandro

(57) **Abstract**

Dietary supplement for sports use, suitable for being taken orally by a user before and/or after a physical or sporting activity, as for example a resistance and/or endurance sport, such as running, mountain running, marathon, cycling, activities that combine multiple disciplines, such as triathlon, trail, ultra-trail, mountain trail, etc., wherein the dietary supplement includes carbohydrates, amino acids and/or proteins, including for example L-glutamine, caffeine, at least one B vitamin, including vitamins B1 and B3; kit comprising a first dietary supplement to be taken before a physical or sporting activity and a second dietary supplement to be taken after a physical or sporting activity, for example for a resistance and/or endurance sport.

## Description

### TECHNICAL FIELD OF THE INVENTION

The present invention refers to a dietary supplement for sports use.

More specifically, the present invention refers to a dietary supplement for use before and/or after resistance and/or endurance sports, aimed at improving the athletic performance of a user and/or recovering energy and nutrients spent on the execution of this sport.

### STATE OF THE PRIOR ART

The percentage of people who practice a sport continuously or occasionally is in continuous increase. Among these people there are sportsmen of all ages who practice the most varied activities.

In recent years, however, there have been an increase in resistance and endurance sports, such as running, mountain running, marathon, cycling and activities that combine multiple disciplines such as triathlon.

The ever-increasing number of events organized in the field, such as trail, ultra-trail and mountain trail, as well as bicycle races, shows how the number of enthusiasts who are constantly dedicated to these activities is constantly increasing.

Endurance sports require that who practices them take care of their diet in a particular and functional way, in that there is a need of energy both before and during and also after the execution of these activities, in order to reach their goals or simply to finish a race.

For this reason, there are many products on the market aimed at those involves in endurance sports, which are divided mainly into three main categories, based on the moment of use: products to be used before, during or after sporting activity.

The products to be used before physical or sporting activity (pre-workout) may include supplements containing carbohydrates for the body, especially slow-release carbohydrates. Since glucides are the main source of energy for the body thanks to glucose which promotes the increase of blood sugars and causes the increase of the energy level. Slow-release carbohydrates allow energy to be released to the body constantly for a prolonged period of time, ideally during the entire duration of the effort that the athlete has to make.

Another very common solution is that of pre-workout products that contain branched-chain amino acids or BCAAs.

Branched chain amino acids are a group of three essential amino acids comprising a branched side chain, respectively L-Leucine, L-Isoleucine and L-Valine. This type of amino acids is found in many foods, especially of animal origin (meat, fish and in particular milk derivatives) and has assumed a considerable importance in the world of sporting nutritional supplementation due to their high presence within the proteins that form the muscles of our body, due to the absence of noteworthy side effects and due to the potential ergogenic and myoprotective role. Furthermore, BCAAs have always had a significant clinical relevance for the treatment of certain diseases or following trauma.

In the sports field, they have significant positive aspects: as an energetic substrate before training, as an anti-catabolic remedy, as a prevention for muscle damages, to reduce the sensation of fatigue and to optimize the growth of muscle.

Often the amino acids are accompanied by the vitamins of group B (B1, B2, B3, B5, B6, B8, B9 and B12), which are part of the water-soluble vitamins that perform an important metabolic function and therefore are essential in the diet of every sporty.

The products to be used during physical or sporting activity (workout) are mainly used to replenish the fluids, vitamins and mineral salts lost during physical activity. Therefore, they are mainly in liquid form to provide for this need and they include vitamins and mineral salts. Furthermore, in endurance sports, these products also comprise carbohydrates (especially those with a high and very high glycemic index) to facilitate the recovery of the consumed energy. These carbohydrates are both rapidly absorption (to immediately provide the necessary energy) and slow release, in order to reach the end of physical effort.

Finally, the products to be used after sports activity (post-workout) have the main purpose of facilitating recovery after physical effort, thus reintroducing the consumed nutrients and repairing any muscle damages. These products are used to re-hydrate and introduce into own body vitamins and mineral salts that have been eliminated with sweat during exertion. These are therefore still supplements in liquid form comprising vitamins (especially group B, which fight tiredness, stress and fatigue, promoting the recovery after an intense workout) and mineral salts or carbohydrates with rapid absorption and slow release to return at the standard the glycogen levels.

Other products of this type may include proteins derived from milk (such as casein, which ensures the body a constant and gradual release of proteins over time, and whey, which provides an immediate protein intake), in that they help athletes to protect their muscles from prolonged physical stress.

However, there is a need for a dietary supplement that is complete, which aims at improving performance for resistance and/or endurance sports activities, as well as helping the nutritional and energetic recovery after exertion; there is also a need for a kit comprising a first dietary supplement designed for taking before physical or sporting activity (pre-activity or pre-workout) and a second supplement designed for taking after physical activity sports (post-activity or post-workout).

### AIMS OF THE INVENTION

The present invention has the technical task of improving the state of the art in the field of dietary supplements for sports use, especially dedicated to sports that require high resistance and/or long endurance.

As part of this technical task, an aim of the present invention is to provide a dietary supplement that allows to improve the athletic performance of the user during physical or sporting activity.

A further object of the present invention is to provide a dietary supplement that can be used before and/or after physical or sporting activity, in order to prepare the user to carry out this activity and/or to recover energy and the consumed substances during the execution of the activity itself.

A still further object of the present invention is to provide a dietary supplement which is ready instantly, without the need to be cooked or long preparation steps, and which is complete, in the sense that it includes all those components or ingredients in a single product, which the user needs before performing and/or after having performed physical or sporting activity.

According to an aspect of the present invention, a dietary supplement according to claim 1 is provided.

A further advantage of the present invention is that of providing a kit comprising a first dietary supplement designed to be taken before physical or sporting activity (pre-activity or pre-workout), capable of supporting performance, and a second supplement designed to be taken after physical or sporting activity (post-activity or post-workout), to better reintegrate what the body has consumed and to assist in the recovery of the body.

A still further advantage of the invention is to provide a kit, wherein there are a first and a second dietary supplement, which therefore have a double formulation, each specific for the moment in which the supplement must be assumed with respect to the execution of physical or sporting activity.

A further advantage of the invention is that of providing a kit, wherein there are a first and a second dietary supplement, each of which has a complete formulation, enclosing in a single product all those components or ingredients that the user needs before performing and/or after performing physical or sports activity.

According to an aspect of the present invention, a kit is provided comprising a first dietary supplement and a second dietary supplement according to claim 14.

The dependent claims refer to preferred and advantageous forms of the invention.

### EMBODIMENTS OF THE INVENTION

The present invention relates to a dietary supplement, suitable for being taken by a user for the performance of a physical or sporting activity, such as for example a physical or sporting activity of resistance and/or endurance. The high resistance and/or endurance activities are, for example, running, trail running, ultra-trail running, mountain trail running or mountain running, marathon, cycling, activities that combine multiple disciplines such as triathlon, etc.

The dietary supplement according to the invention can be taken orally before and/or after physical or sports activity.

In at least one version of the invention, the dietary supplement is in the form of powder. In another version, the dietary supplement is in the form of a drink. In the drink, the powder can be dissolved or diluted in water or other liquid food (for example orange juice, tea, coffee, fruit and/or vegetable smoothie, another dietary supplement based on mineral salts in liquid form, etc.).

In the event that the dietary supplement is in powder form, it can be easily prepared before use by dissolving and/or mixing the powder in the appropriate liquid chosen by the user. If it is in the form of a drink, it is ready for use and therefore immediately usable by the user. This allows the user to prepare the dietary supplement quickly and easily, without the need to cook or to carry out long or complicated steps of preparation of the same.

One of the peculiarities of the invention is to provide a complete dietary supplement, that is, it includes all those components or ingredients that the user needs, respectively, before performing physical or sports activity or after performing the same.

Indeed, the supplements on the market often include only one type of ingredients, for example only carbohydrates or only amino acids/proteins, possibly in combination with vitamins or mineral salts. The dietary supplement according to the present invention, instead, includes all those ingredients or components that allow to improve performance (and therefore they are helpful to the user before activity) or that allow him/her to recover after exertion (at high intensity and/or endurance, as mentioned), and which therefore they are helpful to him after the activity.

The dietary supplement according to the invention, therefore, includes types of different ingredients but all useful for the specific activity to be carried out, as said endurance activity, for example, declined according to the moment in which this supplement must be taken by the user.

Furthermore, in the dietary supplement according to the present invention, each ingredient is selected to be the one that best performs the respective function for the user, without weighing him/her down or causing deficiencies. Therefore, the formulation of the dietary supplement is complete and combines the "right" type of carbohydrates (in a version of the invention different in the pre-activity and in the post-activity), the "right" type of proteins and/or amino acids (in a different version in the pre-activity and in the post-activity), in addition to caffeine, vitamins of group B.

On a general level, indeed, the dietary supplement according to the present invention comprises carbohydrates, amino acids and/or proteins, including L-glutamine, caffeine and at least one vitamin of group B, including for example vitamins B1 and B3.

The carbohydrates present in the dietary supplement according to the present invention may comprise maltodextrin, isomaltulose, trehalose or mixtures thereof.

For example, the dietary supplement comprises carbohydrates in an amount comprised between 50 and 75 grams, or preferably 75 grams, in 100 grams of dietary supplement. For example, the dietary supplement comprises L-glutamine in an amount comprised between 1.5 and 4 grams, or preferably 3.4 grams, in 100 grams of dietary supplement and/or comprises caffeine in an amount comprised between 80 mg and 400 mg, or preferably 80 mg, in 100 grams of dietary supplement.

For example, the dietary supplement comprises at least one vitamin of group B, which is present at 100% of the Nutrient Reference Values (NRVs).

The dietary supplement according to the present invention, in a first version, for example to be used before physical or sporting activity, comprises as said carbohydrates, amino acids and/or proteins, including L-glutamine, caffeine, at least one vitamin of the group B, including vitamins B3, B1, B12 and B9, as well as L-arginine, citrulline, creatine, spirulina and/or moringa oleifera, beta-alanine, and N-acetyl carnitine.

In at least one version of the invention, the dietary supplement according to the invention comprises single amino acids at the moment in a first version, wherein it is a dietary supplement to be taken before physical or sports activity (pre-workout), while it comprises proteins in a second version, when it is a dietary supplement to be taken after physical or sports activity (post-workout).

Maltodextrins guarantee excellent absorption and the absence of fibers, fats, proteins, and anti-nutritional substances.

Maltodextrins are water-soluble complex carbohydrates obtained through chemical processes of hydrolysis mainly from the breakdown of starches from cereals (corn, oats, wheat, rice) or tubers (potatoes, tapioca). Based on the degree of transformation of these starches, maltodextrins are created, consisting of glucose molecules arranged approximately in long polymer chains. The length of the latter provides the parameter that allows to identify and classify maltodextrins based on their dextrose-equivalence (DE) starting from 2-4 up to 19. The higher the DE, the shorter the polysaccharide chain, therefore maltodextrin will have a digestive behavior more similar to that of glucose.

Dextrose-equivalence (DE) can therefore also identify the time of energy release in the human body.

The maltodextrins present in a version of the dietary supplement, in particular used pre-activity, have the characteristic of presenting a prolonged release of energy over time and therefore are maltodextrins with medium-low dextrose-equivalence (for example DE = 12), which guarantee a more regular maintenance of glycaemia without leading to an excessive initial peak of energy, which would not be able to support physical activity in the following hours after consumption. In this way, maltodextrins are able to guarantee an adequate energy supply for the entire duration of the athletic gesture.

Isomaltulose is a disaccharide that is commercially produced through bacterial fermentation starting from saccharose. In nature, it is found in honey and cane sugar. Isomaltulose is completely absorbed in the small intestine like glucose and fructose; it is completely digested, like saccharose, and provides approximately the same caloric count of 4 kcal/g. However, it has a low insulin and glycemic index. Isomaltulose allows glucose to let into the blood slowly, avoiding peaks and sudden drops in glucose levels and consequently in insulin levels. This allows a balanced and prolonged energy supply, in the form of glucose. Furthermore, it promotes the amount of fat used as energy, increasing performance during prolonged efforts.

Trehalose is a disaccharide that has a glycosidic (acetal) bond by condensation between two glucose molecules; it is widespread in yeasts, mushrooms, and insects.

The amino acids contained in the dietary supplement according to the present invention, they are able to play a key role in metabolic processes for the availability of energy. In this way, they play a role consistent with physical effort, without unnecessarily burdening the digestion of the athlete. Indeed, during prolonged effort activities, protein synthesis is practically null, in favor of the catabolic activity on the muscles themselves.

L-glutamine is the most abundant amino acid in the human body; it is widely used during physical activity, so its need increases significantly in the case of intense exercise. Indeed, L-glutamine increases the availability of muscle glycogen during recovery. Furthermore, it is believed that L-glutamine also has an ergogenic effect, which promotes recovery, optimizes protein synthesis and exhibits an anti-catabolic effect, as well as an adjuvant effect on the immune system.

In the event that the dietary supplement is pre-workout, it should be taken about 1 hour before physical activity, since carbohydrates and L-glutamine ensure optimal absorption that supports the athletic movement.

L-arginine is another amino acid that plays an important role in endurance sports, especially in that it is the precursor of nitric oxide (NO).

NO is a molecule with remarkable properties: it is a vasodilator, an anticoagulant, a neurotransmitter, a promoter of angiogenesis (i.e. it promotes the formation of new blood vessels), an anti-inflammatory (it decreases chronic pain), it promotes more rapid the removal of lactate, promotes the synthesis of new mitochondria and mitochondrial enzymes, etc. Furthermore, L-arginine is used to support physical or mental stress, to support the immune system (increases the number of white blood cells, increases the secretion of GH growth hormone, increases noradrenaline), to treat trauma, since it stimulates the synthesis of collagen and promotes rapid scarring, to counteract erectile dysfunction, to help the development of tissues and muscle mass, to decrease blood pressure, etc.

Citrulline is an alpha-amino acid. The endogenous synthesis of L-arginine occurs only in the kidneys starting from citrulline which is released in the small intestine.

Furthermore, since citrulline, taken orally, does not have systemic and pre-systemic elimination forms, its intake allows good levels of arginine and citrulline. On the contrary, arginine undergoes intense pre-systemic metabolism, especially by intestinal arginase, which converts arginine into ornithine and urea, and by hepatic arginases. Therefore, the fact that the dietary supplement according to the invention comprises both L-glutamine and citrulline allows to optimize metabolic availability.

Caffeine increases muscle endurance and muscle strength by promoting the production of glucose with glycogenolysis and by encouraging the use of fats by saving glycogen reserves.

Creatine is especially indicated to improve the intense, short and repeated expressions of strength, but it is also useful for endurance activities to cover those anaerobic phases that may arise.

Spirulina is an iron supplement, so it helps to oxygenate and fluidify the blood, it is an antioxidant, helping for example to eliminate metabolic waste products and avoid muscle inflammation, as well as reducing the recovery times of physical stress.

In addition, it is a source of vegetable proteins that provide essential amino acids, increases the resistance to exertion (fatigue times become longer, the oxidation rate of carbohydrates decreases and the oxidation rate of fats increases) and is a source of vitamins B1, B2, B3, B6, B12, vitamin E and essential fatty acids.

Spirulina is also an excellent support for the immune system and, therefore, a valid ally to avoid possible injuries.

Moringa oleifera has a complete protein content, i.e. the parts of the plant contain the all range of amino acids required for protein needs, even the essential ones. The leaves are very rich in proteins, vitamins, and minerals.

The vitamins of group B (in particular B1, B3, B12 and B9/folic acid) play an important role, since they are important coenzymes for energy metabolism. Folic acid is associated with vitamin B12 for its anti-anemic function. Folic acid also is helpful to the production of hemoglobin, participates in the synthesis of some amino acids, is essential for the proper functioning of the immune system, and is necessary for athletes.

N-acetyl carnitine is a quaternary ammonium, derived from two amino acids, lysine and methionine, which allows both long-chain fatty acids to enter into mitochondria to be oxidized in order to produce ATP, and to cleanse the mitochondria (from acetyl groups and acrylic groups that are formed). N-acetyl carnitine is used metabolically mainly at the level of skeletal muscle and heart muscle; its presence leads to an increase in muscle mass, a decrease in physical and mental fatigue, a better athletic performance and a better recovery.

Beta-alanine is a natural amino acid, which is not found in foods by itself. It is a component of peptides such as carnosine, anserine and balenine, present in meat and fish.

**Table 1: Example of ingredients of a version of the dietary supplement (on a portion of 95-100 gr)**

| **Ingredient** | **Quantity** | **Possible quantity range** |
|---|---|---|
| Maltodextrin (example with DE 12) | 75 g | from 50 g to 75 g |
| Creatine | 5 g | from 3 g to 5 g |
| L-Citrulline | 4 g | from 4 g to 10 g |
| L-Glutamine | 3,4 g | from 1,5 g to 4 g |
| L-arginine | 2,8 g | between 2 g and 10 g |
| Spirulina | 2 g | between 1 g and 5 g |
| N-acetyl carnitine | 1,2 g | between 0,5 g and 2 g |
| Beta-alanine | 800 mg | between 0,8 g and 6 g |
| Caffeine | 80mg | 80 mg and 400 mg |
| B3 | 16 mg 100% NRVs | |
| B1 | 1,1 mg 100% NRVs | |
| B12 | 2,5 mcg 100% NRVs | |
| B9 (folic acid) | 200 mcg 100% NRVs | |

| | | |
|---|---|---|
| NRVs = Nutrient Reference Values; 1 mcg = 10-4 centigram | | |

In one version of the invention, the list in Table 1 corresponds to the formulation of a dietary supplement to be taken before physical or sporting activity (pre-activity or pre-5 workout).

As mentioned, in a second version of the invention, the dietary supplement can also be used after physical or sporting activity (post-activity or post-workout).

In this version, the dietary supplement comprises as mentioned carbohydrates, amino acids and/or proteins, including L-glutamine, caffeine, at least one vitamin of group B, including for example vitamins B3, B1, B2 and B6, and also L-leucine.

Unlike the training phase wherein protein synthesis is practically null, in the recovery phase (post-activity or post-workout) it is essential both to restore glycogen reserves and to ensure muscle recovery.

In detail, insulin, after being stimulated by a correct intake of carbohydrates and proteins, will favor glycogen-synthesis, enzymes and anabolic hormones require protein material to carry out their restorative and damage action due to physical exercise, finally in endurance exercises the ideal ratio (CHO: Pr) of use of carbohydrates (CHO) and proteins (Pr) is 4:1 (0.8-1 g/kg h of CHO and 0.2-0.4g/kg/h of Pr).

For this reason, in this version of the invention the dietary supplement includes, among the carbohydrates, maltodextrins, which however - unlike the formulation of the pre-activity dietary supplement - are maltodextrins with high dextrose-equivalence (DE) to favor the rapid recovery of glycogen stores, such as maltodextrin with DE = 19.

As mentioned, more proteins than amino acids can be present in this version. After physical activity, in fact, the organism has time to digest proteins, and therefore it is not necessary to directly supply the single amino acids.

The proteins present in the dietary supplement according to the present invention may comprises: whey protein, possibly in concentrated form, or isolated whey protein, isolated protein from vegetal foods, such as rice and/or pea, non-isolated proteins, i.e. deriving from a food of plant or animal origin, or a combination thereof.

Compared to isolated proteins, concentrated proteins have a lower degree of purity and comprise lactose which provides a certain amount of carbohydrates (in addition to creating problems for the intolerant).

Isolated whey proteins are obtained through hydrolysis process and have the advantage of not containing lactose and providing a higher percentage of proteins.

The isolated proteins from plant sources have a degree of purity that can reach up to 90%. These are hydrolyzed proteins, i.e. already broken down for which they come absorbed faster.

Rice and/or pea are two vegetal sources or vegetal foods that complete each other in terms of amino acid content. Therefore, their eventual combination provides the dietary supplement of the invention with a complete amino acid profile and a high Chemical Index (IC).

Non-isolated proteins derive from a real ingredient or food, of vegetable or animal origin, and therefore are not isolated from it. In this case, the ingredient or food will provide mostly proteins, but also other macronutrients, depending on the type of chosen ingredient or food. Such ingredients or foods may have a high protein content and/or an excellent amino acid profile.

Non-isolated proteins derived from at least one of spirulina, goji flour, moringa flour, or combinations thereof. Therefore, the ingredient or food is at least one of spirulina, goji flour, moringa flour, or combinations thereof.

As for L-glutamine and caffeine, they are also useful in post-workout or post-activity, the latter for its possible effects on the synthesis of muscle glycogen.

L-leucine is an amino acid which, if taken immediately after training, increases protein synthesis of 33%. This effect is linked to the fact that the m-Tor pathway is activated, which stimulates protein synthesis and prevents muscle catabolism (due to the inhibition of the AMPK enzymatic pathway).

The B vitamins are also useful both in pre- and post-activity. In the latter case, especially vitamins B1, B2, B3 and B6 (with anti-inflammatory and antioxidant effect).

**Table 2: Example of ingredients of a version of the dietary supplement (on a portion of 95-100 grams)**

| **Ingredient** | **Quantity** | **Possible quantity range** |
|---|---|---|
| Maltodextrin (ex. with DE 19) | 75 g | from 50 g to 75 g |
| Isolated pea proteins | 10 g | between 8 g and 15 g |
| Isolated rice proteins | 3 g | between 2 g and 75 g |
| L-glutamine | 3,4 g | from 1,5 g to 4 g |
| L-leucine | 3,1 g | between 2 g and 4 g |
| Caffeine | 80 mg | 80 mg and 400 mg |
| B3 | 16 mg 100% NRVs | |
| B1 | 1,1 mg 100% NRVs | |
| B2 | 1,4 mg 100% NRVs | |
| B6 | 1,4 mg 100% NRVs | |

| | | |
|---|---|---|
| NRVs = Nutrient Reference Values; 1 mcg = 10⁻⁴ centigram | | |

In one version of the invention, the list in Table 2 corresponds to the formulation of a dietary supplement to be taken after physical or sporting activity (post-activity or post-workout).

Certainly, the above indicated tables are only indicative and may be subject to variations.

Optionally, the dietary supplement according to the present invention may also contain mineral salts.

The present invention also refers to a kit comprising a first dietary supplement, to be taken before physical or sporting activity, and a second dietary supplement, to be taken after physical or sporting activity. According to a version of the invention, the first dietary supplement corresponds to that indicated in Table 1 or to the dietary supplement of the first version of the invention, while the second dietary supplement corresponds to that indicated in Table 2 or to the dietary supplement of the second version of the invention.

The first dietary supplement is able to provide the user in a complete way with everything that is useful to take about 1 hour before physical or sporting activity, i.e. macro- and micro-nutrients that improve sports performance.

The second dietary supplement is able to provide the user in a complete way with everything that is useful to take within about 30 minutes of the end of physical or sporting activity, i.e. macro- and micro-nutrients that allow physical recovery post-activity, as well the integration of substances consumed during the activity itself.

It has thus been seen how the present invention can adapt to the specific needs of the user based on certain physical or sports activities that require high resistance and/or long duration, providing a dietary supplement of easy and practical use, which can be taken both before and after the activity.

Described features for an embodiment or variant may also be present in other embodiments or variants, without thereby overcoming the scope of protection determined by the attached claims.

The dietary formulation according to the present invention is susceptible to numerous modifications and variations within the scope of the protection of the following claims. The present invention also relates to the following points, for each of which the Applicant reserves the possibility to file a divisional patent application.
16. Kit according to claim 14 or 15, wherein said second dietary supplement comprises said proteins, which comprise at least one of whey proteins, isolated whey proteins, whey proteins in concentrated form, isolated proteins from vegetal foods, such as rice and/or pea, non-isolated proteins, that is deriving from a food of vegetal or animal origin, such as for example spirulina, goji flour, moringa flour, or combinations thereof.
17. Kit according to any one of the preceding claims from 14 to 16, wherein said carbohydrates are present in an amount comprised between 50 and 75 grams, or preferably 75 grams, in 100 grams of dietary supplement and/or wherein said L-glutamine is present in an amount comprised between 1.5 and 4 grams, or preferably 3.4 grams, in 100 grams of dietary supplement and/or wherein said caffeine is present in an amount comprised between 80 mg and 400 mg, or preferably 80 mg, in 100 grams of dietary supplement and/or wherein said at least one vitamin of group B is present at 100% of the Nutrient Reference Values (NRVs), and/or wherein said vitamin B3 is present in an amount of 16 mg and/or equal to 100% of the Nutrient Reference Values (NRVs), in 100 grams of dietary supplement and/or wherein said vitamin B1 is present in an amount of 1.1 mg and/or equal to 100% of the Nutrient Reference Values (NRVs), in 100 grams of dietary supplement.
18. Kit according to any one of the preceding claims from 14 to 17, wherein said creatine is present in an amount comprised between 3 and 5 grams, or preferably 5 grams, in 100 grams of dietary supplement and/or wherein said L-citrulline it is present in an amount comprised between 4 and 10 grams, or preferably 4 grams, in 100 grams of dietary supplement and/or wherein said L-arginine is present in an amount comprised between 2 and 10 grams, or preferably 2.8 grams, in 100 grams of dietary supplement and/or wherein said spirulina is present in an amount comprised between 1 and 5 grams, or preferably 2 grams, in 100 grams of dietary supplement, and/or wherein said N-acetyl carnitine is present in an amount comprised between 0.5 and 2 grams, or preferably 1.2 grams, in 100 grams of dietary supplement, and/or wherein said beta-alanine is present in an amount comprised between 0.8 and 6 grams, or preferably 0.8 grams, in 100 grams of dietary supplement, and/or wherein said vitamin B12 is present in an amount of 2.5 mcg and/or equal to 100% of the Nutrient Reference Values (NRVs), in 100 grams of dietary supplement, and/or wherein said vitamin B9 is present in a quantity of 200 mcg and/or equal to 100% of the Nutrient Reference Values (NRVs), in 100 grams of dietary supplement.
19. Kit according to any one of the preceding claims from 14 to 18, wherein said isolated pea proteins are present in an amount comprised between 8 and 15 grams, or preferably 10 grams, in 100 grams of dietary supplement, and/or wherein said isolated rice proteins are present in an amount comprised between 2 and 6 grams, or preferably 3 grams, in 100 grams of dietary supplement, and/or wherein said L-leucine is present in an amount comprised between 2 and 5 grams, or preferably 3.1 grams, in 100 grams of dietary supplement, and/or wherein said vitamin B2 is present in an amount of 1.2 mg and/or equal to 100% of the Nutrient Reference Values (NRVs), in 100 grams of dietary supplement, and/or wherein said vitamin B6 is present in an amount of 1.4 mg and/or equal to 100% of the Nutrient Reference Values (NRVs), in 100 grams of dietary supplement.

## Claims

1. Dietary supplement for sports use, suitable for being taken orally by a user before and/or after a physical or sporting activity, such as an endurance and/or duration sport (endurance) such as running, mountain running, the marathon, the cycling, activities that combine multiple disciplines such as triathlon, trail, ultra-trail, mountain trail, etc., **characterized in that** said dietary supplement comprises carbohydrates, amino acids and/or proteins, including for example L-glutamine, caffeine and at least one vitamin of group B, including vitamins B1 and B3.

2. Dietary supplement according to claim 1, wherein said dietary supplement is a first dietary supplement to be taken before said physical or sporting activity.

3. Dietary supplement according to claim 1, wherein said dietary supplement is a second dietary supplement to be taken after said physical or sports activity.

4. Dietary supplement according to any one of the preceding claims when dependent on claim 1 or 2, wherein said carbohydrates comprise at least one of maltodextrin, isomaltulose and trehalose or mixtures thereof and/or in wherein said maltodextrins are of medium-low dextrose-equivalence (DE), for example DE = 12, so as to allow in use a prolonged release of energy over time.

5. Dietary supplement according to any one of the preceding claims when dependent on claim 2, further comprising at least one or more of L-arginine, citrulline, creatine, spirulina and/or moringa oleifera, and N-acetyl carnitine, beta alanine, and wherein said at least one vitamin of group B also comprises vitamins B12 and B9.

6. Dietary supplement according to any one of the preceding claims when dependent on claim 1 or 3, wherein said carbohydrates comprise at least one of maltodextrin, isomaltulose and trehalose or mixtures thereof and/or wherein said maltodextrins are of high dextrose-equivalence (DE), for example DE = 19, so as to favor in use the rapid recovery of glycogen and/or energy reserves.

7. Dietary supplement according to any one of the preceding claims when dependent on claim 3, wherein said proteins comprise at least one of whey proteins, isolated whey protein, whey protein in concentrated form, isolated protein from vegetal foods, such as rice and/or pea, non-isolated proteins, i.e. deriving from a food of vegetable or animal origin, such as spirulina, goji flour, moringa flour, or combinations thereof.

8. Dietary supplement according to any one of the preceding claims when dependent on claim 3, further comprising one or more of L-leucine, pea and/or rice isolated proteins, and wherein said at least one protein of group B also includes vitamins B2, B6.

9. Dietary supplement according to any one of the preceding claims, wherein said carbohydrates are present in an amount comprised between 50 and 75 grams, or preferably 75 grams, in 100 grams of dietary supplement and/or wherein said L-glutamine is present in an amount comprised from 1.5 to 4 grams, or preferably 3.4 grams, in 100 grams of dietary supplement and/or wherein said caffeine is present in an amount comprised from 80 mg to 400 mg, or preferably 80 mg, in 100 grams of dietary supplement and/or wherein said at least one vitamin of group B is present at 100% of the Nutrient Reference Values (NRVs), and/or wherein said vitamin B3 is present in an amount of 16 mg and/or equal to 100% of the Nutrient Reference Values (NRVs), in 100 grams of dietary supplement and/or wherein said vitamin B1 is present in an amount of 1.1 mg and/or equal to 100% of the Nutrient Reference Values (NRVs), in 100 grams of dietary supplement.

10. Dietary supplement according to any one of the preceding claims when dependent on claim 5, wherein said creatine is present in an amount comprised between 3 and 5 grams, or preferably 5 grams, in 100 grams of dietary supplement and/or wherein said L-citrulline is present in an amount comprised between 4 and 10 grams, or preferably 4 grams, in 100 grams of dietary supplement and/or wherein said L-arginine is present in an amount comprised between 2 and 10 grams, or preferably 2.8 grams, in 100 grams of dietary supplement and/or wherein said spirulina is present in an amount comprised between 1 and 5 grams, or preferably 2 grams, in 100 grams of dietary supplement, and/or wherein said N-acetyl carnitine it is present in an amount comprised between 0.5 and 2 grams, or preferably 1.2 grams, in 100 grams of supplement dietary, and/or wherein said beta-alanine is present in an amount comprised between 0.8 and 6 grams, or preferably 0.8 grams, in 100 grams of dietary supplement, and/or wherein said vitamin B12 is present in an amount of 2.5 mcg and/or equal to 100% of the Nutrient Reference Values (NRVs), in 100 grams of dietary supplement, and/or wherein said vitamin B9 is present in an amount of 200 mcg and/or equal to 100% of the Nutrient Reference Values (NRVs), in 100 grams of dietary supplement.

11. Dietary supplement according to any one of the preceding claims when dependent on claim 8, wherein said isolated pea proteins are present in an amount comprised between 8 and 15 grams, or preferably 10 grams, in 100 grams of dietary supplement, and/or wherein said isolated rice proteins are present in an amount comprised between 2 and 6 grams, or preferably 3 grams, in 100 grams of dietary supplement, and/or wherein said L-leucine is present in an amount comprised between 2 and 5 grams, or preferably 3.1 grams, in 100 grams of dietary supplement, and/or wherein said vitamin B2 is present in an amount of 1.2 mg and/or equal to 100% of the Nutrient Reference Values (NRVs), in 100 grams of dietary supplement, and/or wherein said vitamin B6 is present in an amount of 1.4 mg and/or equal to 100% of the Nutrient Reference Values (NRVs), in 100 grams of dietary supplement.

12. Dietary supplement according to any one of the preceding claims, comprising mineral salts.

13. Dietary supplement according to any one of the preceding claims, wherein said dietary supplement is in the form of powder or drink or wherein said powder is dissolved or diluted in water or other food liquid, for example orange juice, tea, coffee, fruit and/or vegetable smoothie, another dietary supplement based on mineral salts in liquid form, etc.

14. Kit comprising at least one dietary supplement for sports use, such as for example a resistance and/or endurance sport, such as running, mountain running, marathon, cycling, activities that combine multiple disciplines such as triathlon, trail, ultra-trail, mountain trail, etc., wherein said kit includes a first dietary supplement designed to be taken orally by a user before a physical or sporting activity and a second dietary supplement designed to be taken orally by a user after a physical or sporting activity, wherein said first and said second dietary supplement comprise carbohydrates, amino acids and/or proteins, including for example L-glutamine, caffeine, at least one vitamin of group B, including vitamins B1 and B3, **characterized in that** said first dietary supplement further comprises one or more of L-arginine, citrulline, creatine, spirulina and/or moringa oleifera, and N-acetyl carnitine, beta alanine, and wherein said at least one vitamin of group B also includes vitamins B12 and B9, while said second dietary supplement further comprises one or more of L-leucine, and wherein said at least one vitamin of group B also includes vitamins B2, B6.

15. Kit according to claim 14, wherein said carbohydrates of said first dietary supplement and/or of said second dietary supplement comprise at least one of maltodextrin, isomaltulose and trehalose or mixtures thereof and/or wherein said maltodextrins of said first dietary supplement have a medium-low dextrose-equivalence (DE), for example DE=12, while said maltodextrins of said second dietary supplement have a high dextrose-equivalence (DE), for example DE = 19.
